# EUROPEAN PATENT SPECIFICATION

(11) **EP 1 771 127 B1**
(45) Date of publication and mention of the grant of the patent: **05.11.2014**
(21) Application number: 05763776.1
(22) Date of filing: 30.06.2005
(51) Int. Cl.: A61F 2/91

(54) **INTRALUMINAL MEDICAL DEVICE HAVING ASYMETRICAL MEMBERS**
INTRALUMINALE MEDIZINISCHE VORRICHTUNG MIT ASYMMETRISCHEN GLIEDERN
DISPOSITIF MEDICAL INTRALUMINAL A ELEMENTS ASYMETRIQUES

(30) Priority: 30.06.2004 US 584375 P
(43) Date of publication of application: 11.04.2007
(73) Proprietor: Cordis Corporation, Fremont, CA 94555 (US)
(72) Inventor: BURGERMEISTER, Robert, Bridgewater, NJ 08807 (US); DUERIG, Thomas, Fremont, CA 94539 (US); GRISHABER, Randy, Asbury, NJ 08802 (US); MARREY, Ramesh, Basking Ridge, NJ 07920 (US); PARK, Jin, Parsippany, NJ 07054 (US); KREVER, Mathew, Warren, NJ 07059 (US)
(74) Representative: Tunstall, Christopher Stephen
(86) International application number: PCT/US2005/023651
(87) International publication number: WO 2006/005027

(56) References cited:
- EP-A- 1 190 685
- EP-A- 1 378 212
- WO-A-03/030786
- WO-A1-97/40781
- WO-A1-03/055414
- WO-A2-02/26164
- DE-A1- 10 144 430
- US-A1- 2003 204 244
- US-B1- 6 245 101

## Description

### CROSS-REFERENCE TO RELATED APPLICATIONS

This application claims priority pursuant to 35 U.S.C. § 119 (e) to provisional application 60/584,375 filed on June 30, 2004.

### FIELD OF THE INVENTION

This invention relates generally to expandable intraluminal medical devices for use within a body passageway or duct, and more particularly to an optimized stent having asymmetrical strut and loop members.

### BACKGROUND OF THE INVENTION

The use of intraluminal prosthetic devices has been demonstrated to present an alternative to conventional vascular surgery. Intraluminal prosthetic devices are commonly used in the repair of aneurysms, as liners for vessels, or to provide mechanical support and prevent the collapse of stenosed or occluded vessels.

Intraluminal endovascular prosthetics involve the percutaneous insertion of a generally tubular prosthetic device, such as a stent, into a vessel or other tubular structure within the vascular system. The stent is typically delivered to a specific location inside the vascular system in a low profile (pre-deployed) state by a catheter. Once delivered to the desired location, the stent is deployed by expanding the stent into the vessel wall. The expanded stent typically has a diameter that is several times larger than the diameter of the stent in its compressed state. The expansion of the stent may be performed by several methods known in the art, such as by a mechanical expansion device (balloon catheter expansion stent) or by self-expansion.

The ideal stent utilizes a minimum width and wall thickness of the stent members to minimize thrombosis at the stent site after implantation. The ideal stent also possess sufficient hoop strength to resist elastic recoil of the vessel. To fulfill these requirements, many current tubular stents use a multiplicity of circumferential sets of strut members connected by either straight longitudinal connecting connectors or undulating longitudinal connecting connectors.

The circumferential sets of strut members are typically formed from a series of diagonal sections connected to curved or arc sections forming a closed-ring, zig-zag structure. This structure opens up as the stent expands to form the element in the stent that provides structural support for the vessel wall. A single strut member can be thought of as a diagonal section connected to a curved section within one of the circumferential sets of strut members. In current stent designs, these sets of strut members are formed from a single piece of metal having a uniform wall thickness and generally uniform strut width. Similarly, the curved loop members are formed having a generally uniform wall thickness and generally uniform width.

Although the geometry of the stent members may be uniform, the strain experienced by each member under load is not. The "stress" applied to the stent across any cross section is defined as the force per unit area. These dimensions are those of pressure, and are equivalent to energy per unit volume. The stress applied to the stent includes forces experienced by the stent during deployment, and comprises the reactive force per unit area applied against the stent by the vessel wall. The resulting "strain" (deformation) that the stent experiences is defined as the fractional extension perpendicular to the cross section under consideration.

During deployment and in operation, each stent member experiences varying load along its length. In particular, the radial arc members are high in experienced loading compared to the remainder of the structure. When the stent members are all of uniform cross-sectional area, the resultant stress, and thus strain, varies. Accordingly, when a stent has members with a generally uniform cross-section, some stent members will be over designed in regions of lesser induced strain, which invariably results in a stiffer stent. At a minimum, each stent member must be designed to resist failure by having the member size (width and thickness) be sufficient to accommodate the maximum stress and/or strain experienced. Although a stent having strut or arc members with a uniform cross-sectional area will function, when the width of the members are increased to add strength or radio-opacity, the sets of strut members will experience increased stress and/or strain upon expansion. High stress and/or strain can cause cracking of the metal and potential fatigue failure of the stent under the cyclic stress of a beating heart.

Cyclic fatigue failure is particularly important as the heart beats, and hence the arteries "pulse", at typically 70 plus times per minute - some 40 million times per year - necessitating that these devices are designed to last in excess of 10⁸ loading cycles for a 10-year life. Presently, designs are both physically tested and analytically evaluated to ensure acceptable stress and strain levels are achievable based on physiologic loading considerations. This is typically achieved using the traditional stress/strain-life (*S*-*N*) approach, where design and life prediction rely on a combination of numerical stress predictions as well as experimentally-determined relationships between the applied stress or strain and the total life of the component. Fatigue loading for the purpose of this description includes, but is not limited to, axial loading, bending, torsional/twisting loading of the stent, individually and/or in combination. One of skill in the art would understand that other fatigue loading conditions can also be considered using the fatigue methodology described as part of this invention.

Typically, finite-element analysis (FEA) methodologies have been utilized to compute the stresses and/or strains and to analyze fatigue safety of stents for vascular applications within the human body. This traditional stress/strain-life approach to fatigue analysis, however, only considers geometry changes that are uniform in nature in order to achieve an acceptable stress and/or strain state, and does not consider optimization of shape to achieve near uniform stress and/or strain along the structural member. By uniformity of stresses, a uniformity of "fatigue safety factor" is implied. Here fatigue safety factor refers to a numerical function calculated from the mean and alternating stresses measured during the simulated fatigue cycle. In addition, the presence of flaws in the structure or the effect of the propagation of such flaws on stent life are usually not considered. Moreover, optimization of the geometry considering flaws in the stent structure or the effect of the propagation of such flaws has not been implemented.

What is needed is a stent design where the structural members experience near uniform stress and/or strain along the member, thereby maximizing fatigue safety factor and/or minimizing peak strain, and analytical methods to define and optimize the design, both with or without imperfections. One such resulting design contemplates stent members with varying cross-sections to produce a near uniform stress and/or strain for a given loading condition with or without the presence of defects or imperfections.

US 6,245,101 B1 discloses an expandable stent having struts which joined together at a node by hinges. The shapes of the struts, hinges and nodes are adapted such that the stent is able to be expanded mechanically or by a balloon, but withstand crush impacts.

EP 1 190 685 A2 and WO 03/055414 also disclose a stent.

### SUMMARY OF THE INVENTION

The present invention provides a stent according to independent claim 1.

This invention relates generally to expandable intraluminal medical devices for use within a body passageway or duct, and more particularly to an optimized stent having asymmetrical strut and loop members. In one embodiment of the invention, the stent comprises one or more hoop components having a tubular configuration with proximal and distal open ends defining a longitudinal axis extending there between. Each hoop component is formed as a continuous series of substantially longitudinally oriented radial strut members and a plurality of radial arc members connecting adjacent radial struts. At least one radial arc member can have non-uniform cross-sections to achieve near-uniform strain distribution along the radial arc when the radial arc undergoes deformation.

Another embodiment of the present invention includes a stent having one or more flex connectors having at least one flex component. The flex component is designed to have non-uniform cross-sections to achieve near-uniform strain distribution along the flex component when the flex component undergoes deformation.

Similarly, another embodiment of the invention the stent comprises one or more radial support members having at least one radial component The radial component is designed to have non-uniform cross-sections to achieve near-uniform strain distribution along the radial component when the radial component undergoes deformation.

In still another embodiment of the invention, the stent comprises one or more members where each member has at least one component. The component is designed to have non-uniform cross-sections to achieve near-uniform strain distribution along the component when the component undergoes deformation.

In still another example, the stent comprises a plurality of hoop components having a tubular configuration with proximal and distal open ends defining a longitudinal axis extending there between. Each hoop component is formed as a continuous series of substantially longitudinally oriented radial strut members and a plurality of radial arc members connecting adjacent radial struts. At least one radial arc member has non-uniform cross-sections to achieve near-uniform strain distribution along the radial arc when the radial arc undergoes deformation. The stent further comprises one or more longitudinally oriented flex connectors connecting adjacent hoop components. Each flex connector comprising flexible struts, with each flexible strut being connected at each end by one flexible arc.

Another stent includes one or more hoop components having a tubular configuration with proximal and distal open ends defining a longitudinal axis extending there between. Each hoop component is formed as a continuous series of substantially longitudinally oriented radial strut members and a plurality of radial arc members connecting adjacent radial struts. At least one radial arc member has non-uniform cross-sections to achieve near-uniform stress distribution along the radial arc when the radial arc undergoes deformation.

A medical device according to the present invention comprises a stent including one or more flex connectors having at least one flex component. The flex component has non-uniform cross-sections to achieve near-uniform stress distribution along the flex component when the flex component undergoes deformation.

The present invention also contemplates a stent having one or more radial support members, including at least one radial component. The radial component has non-uniform cross-sections to achieve near-uniform stress distribution along the radial component when the radial component undergoes deformation.

Another stent according to the present invention comprises one or more members each having at least one component. The component has non-uniform cross-sections to achieve near-uniform stress distribution along the component when the component undergoes deformation.

Still another stent includes a plurality of hoop components having a tubular configuration with proximal and distal open ends defining a longitudinal axis extending there between. Each hoop component is formed as a continuous series of substantially longitudinally oriented radial strut members and a plurality of radial arc members connecting adjacent radial struts. At least one radial arc member has non-uniform cross-sections to achieve near-uniform stress distribution along the radial arc when the radial undergoes deformation. The stent further comprises one or more longitudinally oriented flex connectors connecting adjacent hoop components. Each flex connector comprising flexible struts, with each flexible strut being connected at each end by one flexible arc.

Still another stent comprises one or more hoop components having a tubular configuration with proximal and distal open ends defining a longitudinal axis extending there between. Each hoop component is formed as a continuous series of substantially longitudinally oriented radial strut members and a plurality of radial arc members connecting adjacent radial struts. At least one radial arc member has a non-uniform profile to achieve near-uniform strain distribution along the radial arc when the radial arc undergoes deformation.

The present invention contemplates a stent having one or more flexible connectors connecting adjacent hoop components. Each flexible connector is formed as a continuous series of substantially longitudinally oriented flexible strut members and a plurality of flexible arc members connecting adjacent flexible struts. At least one flexible arc member has a tapered profile to achieve near-uniform strain distribution along the flexible arc when the flexible arc undergoes deformation.

### BRIEF DESCRIPTION OF THE DRAWINGS

Figure 1 is a perspective view of an intraluminal stent in an unexpanded or crimped, pre-deployed condition according to one embodiment of the present invention.
Figure 2 is a perspective view of an intraluminal stent in the fully expanded condition according to one embodiment of the present invention.
Figure 3A is a front view illustrating a stent in its crimped, pre-deployed state as it would appear if it were cut longitudinally and then laid out into a flat in a 2-dimensional configuration according to one embodiment of the present invention.
Figure 3B is a magnified detail view of a proximal hoop element according to one embodiment of the present invention.
Figure 3C is a magnified detail view of an internal hoop element according to one embodiment of the present invention.
Figure 3D is a magnified detail view of a distal hoop element according to one embodiment of the present invention.
Figure 3E is a magnified detail view of a flex connector according to one embodiment of the present invention.
Figure 3F is a magnified detail view of a tapered radial arc according to one embodiment of the present invention.
Figure 4A is a graphical representation of the stress-intensity range (difference in stress intensity factors across the fatigue loads) along the Y-axis versus the length of the discontinuity along the X-axis.
Figure 4B is a graphical representation of Fatigue Life of the stent (along the Y axis) as a function of the discontinuity size (along the X axis)
Figure 5A is a magnified detail view of a stent section as typically found in the prior art.
Figure 5B is a magnified detail view of a stent section according to one embodiment of the present invention.
Figure 5C is a graphical representation of the strain experienced by stent sections at various points along the stent section.

### DETAILED DESCRIPTION OF THE INVENTION

The present invention describes an intraluminal medical device that is capable of expanding into the wall of a vessel lumen and physiological loading, while maintaining near uniform stress (uniform fatigue safety factor) and/or strain in one or more of the device components during use. For the purpose of this description, "use" may include the delivery, deployment and post deployment (short and long term) state of the device. An intravascular stent will be described for the purpose of example. However, as the term is used herein, intraluminal medical device includes but is not limited to any expandable intravascular prosthesis, expandable intraluminal vascular graft, stent, or any other mechanical scaffolding device used to maintain or expand a body passageway. Further, in this regard, the term "body passageway" encompasses any duct within a mammalian's body, or any body vessel including but not limited to any vein, artery, duct, vessel, passageway, trachea, ureters, esophagus, as well as any artificial vessel such as grafts.

The intraluminal device according to the present invention may incorporate any radially expandable stent, including self-expanding stents and mechanically expanded stents. Mechanically expanded stents include, but are not limited to stents that are radially expanded by an expansion member, such as by the expansion of a balloon.

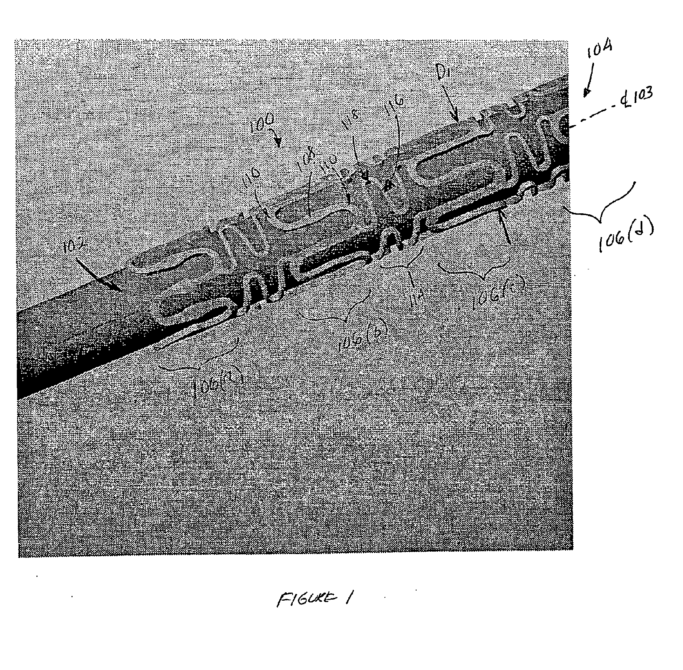

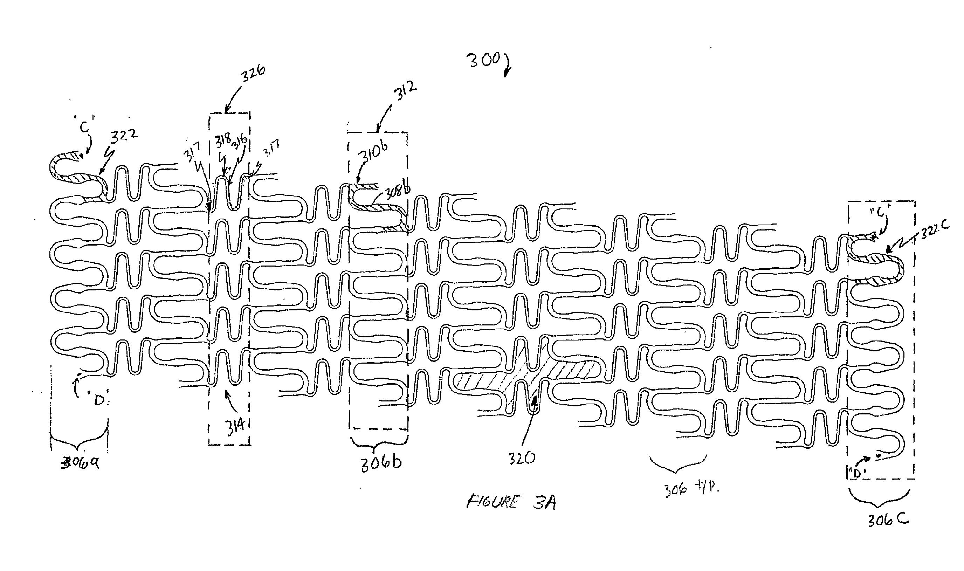

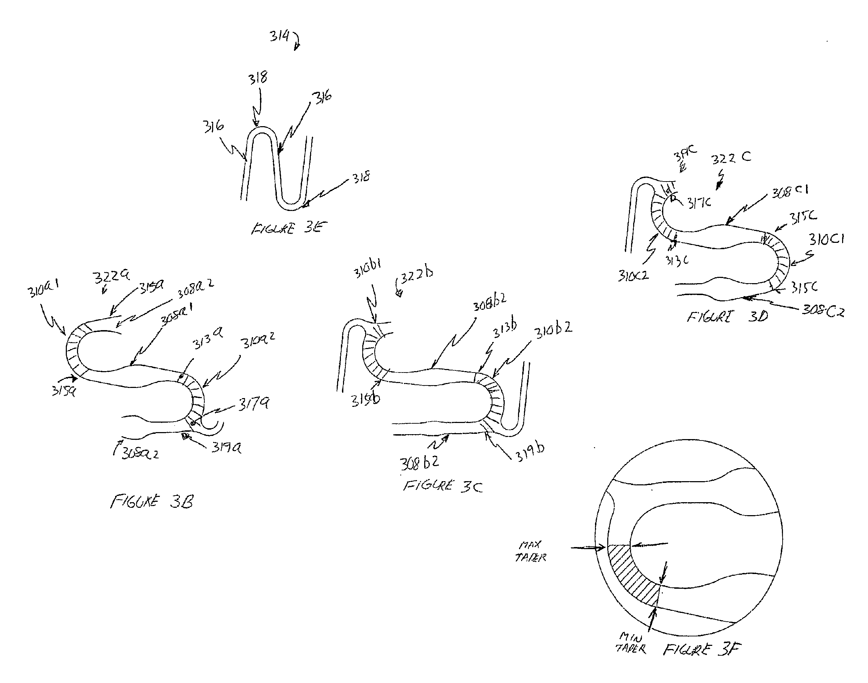

With reference to the drawing figures, like parts are represented by like reference numerals throughout the various different figures. By way of example, radial strut 108 in Figure 1 is similar or equivalent to radial strut 308 in Figure 3.

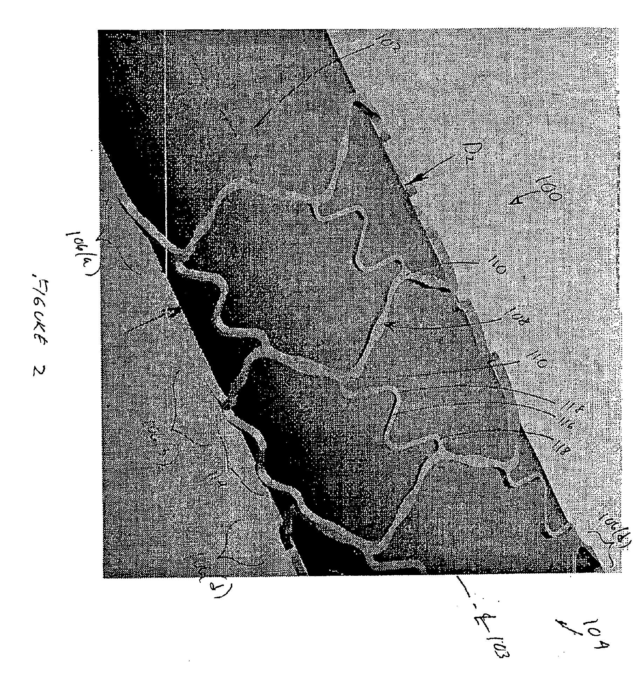

Referring to Figures 1 and 2, there is illustrated perspective views of a stent 100 according to one embodiment of the present invention. Figure 1 illustrates the stent 100 in an unexpanded or crimped, pre-deployed state, while Figures 2 shows the stent 100 in the fully expanded state.

The stent 100 comprises a tubular configuration of structural elements having proximal and distal open ends 102, 104 and defining a longitudinal axis 103 extending there between. The stent 100 has a first diameter D1 for insertion into a patient and navigation through the vessels, and a second diameter D2 for deployment into the target area of a vessel, with the second diameter being greater than the first diameter.

The stent 100 structure comprises a plurality of adjacent hoops 106(a)-(d) extending between the proximal and distal ends 102, 104. In the illustrated embodiment, the hoops 106(a)-(d) encompass various radial support members and/or components. In particular, the radial components that comprise the hoops 106(a)-(d) include a plurality of longitudinally arranged radial strut members 108 and a plurality of radial arc members 110 connecting adjacent radial struts 108. Adjacent radial struts 108 are connected at opposite ends in a substantially S or Z shaped pattern so as to form a plurality of cells. The plurality of radial arc members 110 have a substantially semi-circular configuration and are substantially symmetric about their centers.

The stent 100 structure further comprises a plurality of flex connectors 114, which connect adjacent hoops 106(a)-(d). Each flex connector 114 comprises one or more flexible components. In the embodiment illustrated Figure 1 and 2, the flexible components include one or more longitudinally oriented flexible strut members 116 and a plurality of flexible arc members 118. Adjacent flexible struts 116 are connected at opposite ends in a substantially N shaped pattern. The plurality of flexible arc members 118 have a substantially semi-circular configuration and are substantially symmetric about their centers.

Each flex connector 114 has two ends. One end of the flex connector 114 is attached to one radial arc 110 on one hoop, for examples hoop 106(c), and the other end of the flex connector 114 is attached to one radial arc 110 on an adjacent hoop, for example hoop 106(d). The flex connector 114 connect adjacent hoops 106(a)-(d) together at flex connector to radial arc connection regions 117.

Figures 3A illustrates a stent 300 according to one embodiment of the present invention. The stent 300 is in its crimped, pre-deployed state as it would appear if it were cut longitudinally and then laid out flat in a 2-dimensional configuration. It should be clearly understood that the stent 300 depicted in Figures 3A is in fact cylindrical in shape, similar to stent 100 shown in Figure 1, and is only shown in the flat configuration for the purpose of illustration. This cylindrical shape would be obtained by rolling the flat configuration of Figure 3A into a cylinder with the top points "C" joined to the bottom points "D".

The stent 300 is typically fabricated by laser machining of a cylindrical, Cobalt Chromium alloy tube. Other materials that can be used to fabricate stent 300 include, other non-ferrous alloys, such as Cobalt and Nickel based alloys, Nickel Titanium alloys, stainless steel, other ferrous metal alloys, refractory metals, refractory metal alloys, titanium and titanium based alloys. The stent may also be fabricated from a ceramic or polymer material.

Similar to Figure 1, the stent 300 is comprised of a plurality of cylindrical hoops 306 attached together by a plurality of flex connectors 314. By way of example, a plurality of radial strut members 308b are connected between radial arc members 310b to form a closed, cylindrical, hoop section 306b (as shown within the dotted rectangle 312) in Figure 3A.

A section of flex connectors 314 (as shown within the dotted rectangle 326) bridge adjacent hoop sections 306. Each set of flex connectors 314 can be said to consist of three longitudinally oriented flexible struts 316, with each flexible strut 316 being connected at each end by one of four flexible arc members 318 forming a "N" shaped flexible connector 314 having two ends. Each end of the N flex connector 314 is attached to curved radial arc members 310 at strut flex connector attachment points 317.

In the illustrated embodiment, each hoop section 306 is comprised of radial struts 308 and radial arcs 310 arranged in a largely sinusoidal wave pattern. Each flex connector is attached to the adjacent hoop 306 every complete sinusoidal cycle, such that the number of N flex connectors 314 in the set of N flex connectors 326 is one-half of the total number of radial arc members 310 in the hoop section 306. Figure 3E depicts a detail of a typical flex connector 314 having a longitudinally oriented flexible strut 316 connected at each end to a flexible arc 318.

Each N flex connector 314 is shaped so as to nest together into the adjacent N flex connector 314 as is clearly illustrated in Figure 3A. "Nesting" is defined as having the top of a first flexible connector inserted beyond the bottom of a second flexible connector situated just above that first flexible connector. Similarly, the bottom of the first flexible connector is inserted just below the top of a third flexible connector that is situated just below that first flexible connector. Thus, a stent with nested individual flexible connectors has each individual flexible connector nested into both adjacent flexible connectors; i.e., the flexible connector directly below and the flexible connector directly above that individual flexible connector. This nesting permits crimping of the stent 300 to smaller diameters without having the "N" flex connectors 314 overlap.

Stent 300 illustrated in Figure 3A is comprised of 9 hoop sections 306 connected by 8 sections of flex connectors 314. The 9 hoop sections 306 include 2 end hoop sections (proximal hoop section 306a and distal hoop section 306c) and 7 internal hoop sections 306b.

The internal hoop sections 306b are connected at opposite ends by the sections of flex connectors 314 in a defined pattern to form a plurality of closed cells 320. The end hoop sections (306a and 306c) are connected at one end to the adjacent internal hoop section by a section of flex connectors 314, and similarly form a plurality of closed cells. Adjacent hoop sections 306 may be oriented out of phase, as illustrated in Figure 3A. Alternatively, the adjacent hoop sections 306 may be oriented in phase. It should also be noted that the longitudinal length of the end hoop sections (306a and 306c) may be of a different length than the longitudinal length of the internal hoop sections 306b. In the embodiment illustrated in Figure 3A, the end hoop sections (306a and 306c) have a shorter longitudinal length than the internal hoop sections 306b.

As described above, each hoop section in the illustrated embodiment is comprised of radial strut members 308 and radial arc members 310 arranged in a largely sinusoidal wave pattern. Each repeating wave pattern forms a hoop element 322. The hoop element repeats at each flex connector 314 (in a given set of flex connectors 326) and forms the hoop 306.

By way of example, Figure 3A shows each hoop section 306 being comprised of 5 hoop elements 322. However, the number of repeating hoop elements 322 is not meant to limit the scope of this invention. One of skill in the art would understand that larger and smaller numbers of hoop elements may be used, particularly when providing stents of larger and smaller diameter.

Figures 3B through 3D are magnified detail views of proximal hoop element 322a, internal hoop element 322b, and distal hoop element 322c, respectively, according to an embodiment of the present invention. The proximal end hoop element 322a is attached to the flex connector 314 along its distal end. The distal end hoop element 322c is attached to the flex connector 314 along its proximal end. Figure 3C illustrates a typical internal hoop element 322b attached to adjacent flex connectors 314 along its proximal and distal ends.

As earlier described, hoop element 322 comprises a plurality of radial struts 308 and radial arcs 310 arranged in a largely sinusoidal wave pattern. To achieve uniform stress and/or strain in each element of the wave pattern, the hoop elements 322 are, in general, comprised of radial struts 308 and radial arcs 310 of varying dimensions within each hoop element 322. This design configuration includes radial struts 308 having different cross-sectional areas. In addition, the proximal and distal end hoop elements 322a and 322c are of a different configuration than the internal hoop elements 322b. Accordingly, the radial arcs 310 and radial strut 308 members that are part of the internal hoop element 322b may be a different dimension than the corresponding strut on the proximal or distal end hoop elements 322a and 322c respectively. The proximal and distal hoop elements 322a and 322c are mirror images of one another.

The intravascular stent must be circumferentially rigid and possess sufficient hoop strength to resist vascular recoil, while maintaining longitudinal flexibility. In typical sinusoidal and near sinusoidal designs, the radial arcs experience areas of high stress and/or strain, which are directly related to stent fatigue. However, the stress and/or strain experienced along the length of the radial arc is not uniform, and there are areas of relatively low stress and/or strain. Providing a stent having radial arcs with uniform cross-sectional results in areas of high maximum stress and/or strain and other areas of relatively low stress and/or strain. The consequence of this design is a stent having lower expansion capacity as well as lower fatigue life.

The stent design according to the present invention has been optimized around stress (fatigue safety factor) and/or strain, which results in a stent that has near uniform strain, as well as optimal fatigue performance, along the critical regions of the stent. Optimal fatigue performance is achieved by maximizing the near uniform fatigue safety factor along the stent. Various critical regions may include the radial arcs 310 and/or radial struts 308 and/or flexural arcs 318 and/or flexural struts 316. In a preferred embodiment the critical region includes the radial arc 310. One method of predicting the stress and/or strain state in the structure is finite element analysis (FEA), which utilizes finite elements (discrete locations).

This design provides a stent having greater expansion capacity and increased fatigue life. Where initial stress and/or strain was high, material was added locally to increase the cross-sectional area of the radial arc 310, and thereby distribute the high local stress and/or strain to adjacent areas, lowering the maximum stress and/or strain. In addition, changing the geometry of the cross-section may also result in similar reductions to the maximum stress and/or strain. These techniques, individually or in combination (i.e. adding or removing cross-sectional area and or changing cross-sectional geometry) are applied to the stent component, for example, radial arc 310, until the resultant stress and/or strain is nearly uniform. Another benefit of this design is a stent having reduced mass.

The scope of this invention includes fracture-mechanics based numerical analysis in order to quantitatively evaluate pre-existing discontinuities, including flaws in the stent structure, and thereby predict stent fatigue life. Further, this methodology can be extended to optimize the stent design for maximum fatigue life under the presence of discontinuities. This fracture-mechanics based approach according to the present invention quantitatively assesses the severity of discontinuities in the stent structure including microstructural flaws, in terms of the propensity of the discontinuity to propagate and lead to in vivo failure of the stent when subjected to the cyclic loads within the implanted vessel. Specifically, stress-intensity factors for structural discontinuities of differing length, geometry, and/or position of the discontinuity within and upon the stent structure are characterized, and the difference in the stress intensities associated with the cyclic loads are compared with the fatigue crack-growth thresholds to determine the level of severity of the discontinuity. Experimental data for fatigue crack-growth rates for the stent material are then used to predict stent life based on the loading cycles required to propagate the discontinuity to a critical size.

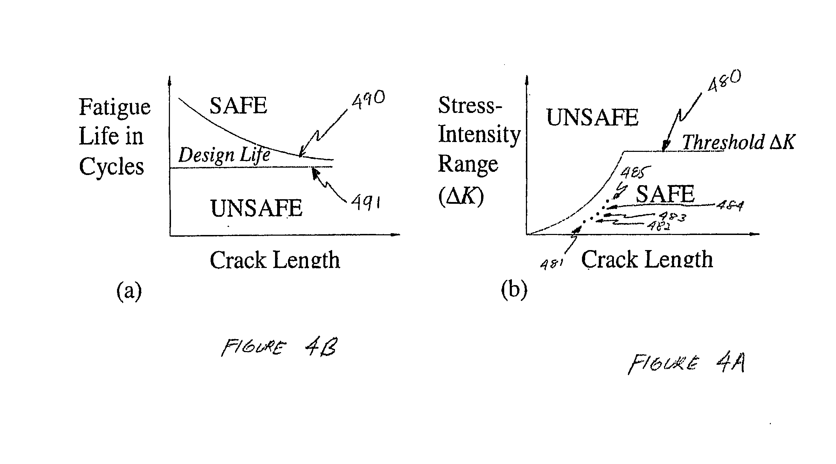

Figure 4A is a graphical representation of the stress-intensity range (difference in stress intensity factors across the fatigue loads) along the Y-axis versus the length of the discontinuity along the X-axis. The solid line 480 represents the threshold stress intensity range depicted as a function of discontinuity length. This threshold stress range is characterized for the given stent material. For a given stent design, discontinuities of differing length, geometry, and/or position of the discontinuity within and upon the stent structure are numerically analyzed by introducing them into and/or onto the stent structure, and the stress intensity ranges are computed for the fatigue loads in question. By way of example, the dotted points 481-485 in Figure 4A represent the calculated stress intensity ranges for various discontinuity lengths. If these points 481-485 fall below the threshold stress intensity curve 480 for a given discontinuity length, the discontinuity is considered unlikely to propagate during stent use, and in particular use during the long term post deployment state. Conversely, if the points 481-481 fall on or above curve 480, the discontinuity is more likely to propagate during use.

A more conservative approach can be achieved by numerically integrating the fatigue crack propagation relationship for the given stent material between the limits of initial and final discontinuity size. This approach disregards the existence of threshold stress intensity range and is therefore considered more conservative. The numerical integration results in predictions of finite lifetimes for the stent as a function of discontinuity size. Figure 4B is a graphical representation of Fatigue Life of the stent (along the Y axis) as a function of the discontinuity size (along the X axis), and is characterized by curve 490.

Curve 490 is compared to the design life of the stent, curve 491, for additional assessment of stent safety. If the predicted fatigue life 490 for a given discontinuity size is greater than the design life 491, stents with these discontinuities are considered safe. Conversely, if the predicted fatigue life 490 for a given discontinuity size is less than or equal to the design life 491, stents with these discontinuities are considered more susceptible to failure during use.

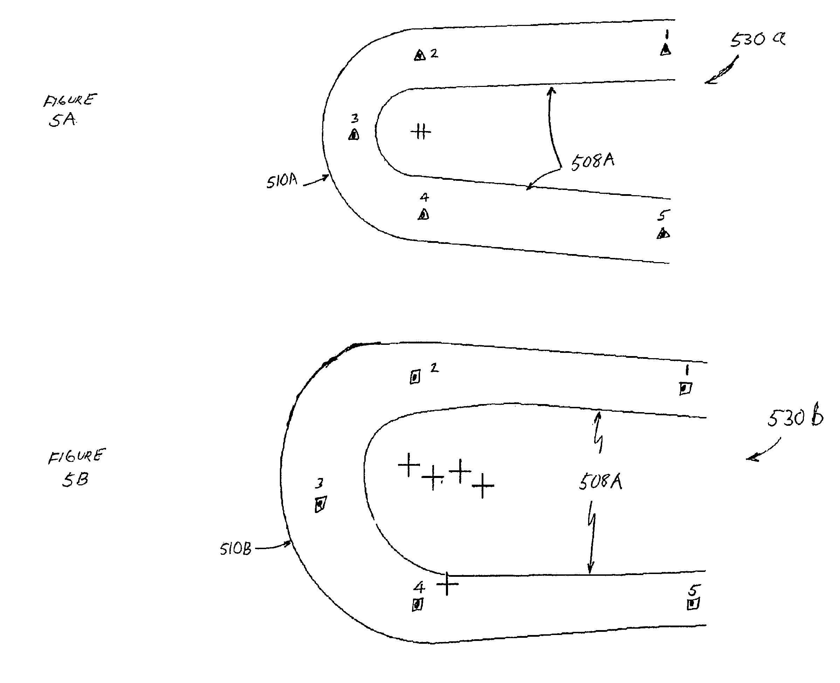

Figures 5A through 5C may be used to compare the strain experienced by the stent according to one embodiment of the present invention to a typical prior art stent configuration. Figure 5A shows a magnified detail view of a radial arc 510a and adjacent radial struts 508a (hereinafter stent section 530a) for a prior art stent. As can be seen in the illustrated section 530a, the radial arc 510a has a uniform width along its entire length.

Figure 5B shows a similar magnified detail view of a radial arc 510b and adjacent radial struts 508b (hereinafter stent section 430b) for a stent according to one embodiment of the present invention. Unlike the prior art stent section 530a shown in Figure 5A, the radial arc 510b has a non-uniform width to achieve near uniform strain throughout the radial arc 510b.

In this description, strain optimization is being described for the purpose of example. However, one of skill in the art would understand that this method may also be applicable to optimize the stress state as well.

For comparative purposes, the strain at five position points (1 through 5) along each illustrated stent section 530 was measured for a given expansion diameter. Position point 1 is located along the radial strut 508. Position points 2 and 4 are located at each root end of the radial arc 510, where the radial arc 410 connects to the radial strut 508. Position point 3 is located along the radial arc 510 at or near the apex or radial midpoint.

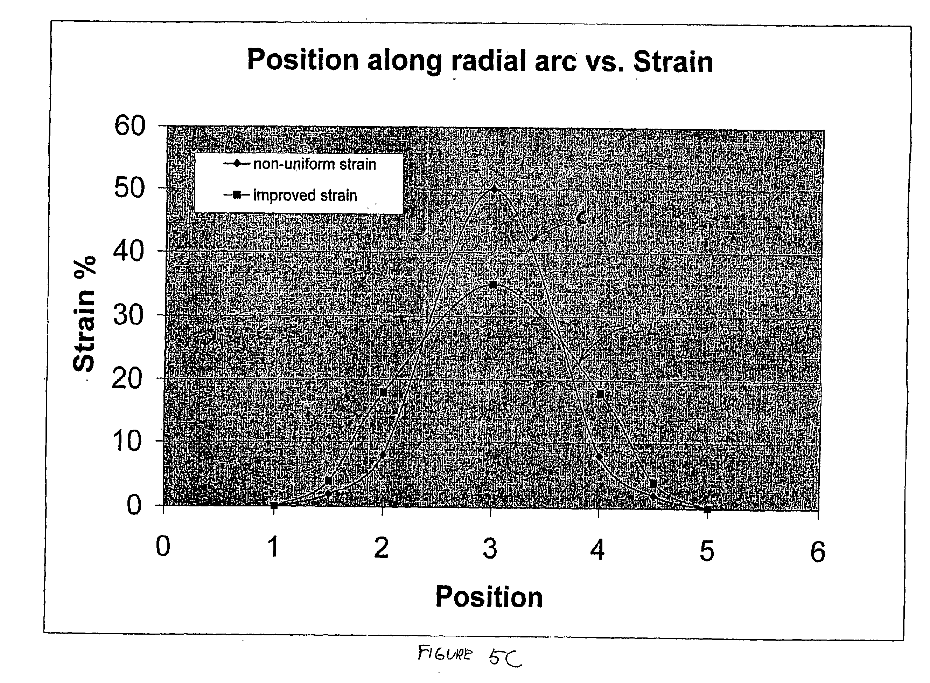

A graphical representation comparing the strain experienced by the prior art stent section 530a to the strain experienced by the stent section 530b for a given expansion diameter is illustrated in Figure 5C. The strain experienced by the prior art stent is identified in the graph by curve C1, having non-uniform strain, with the strain position points designated by a diamond shape. The total strain experienced by the prior art sent section 530a is the area under the curve C1.

The strain experienced by the stent according to one embodiment of the present invention is identified in the graph by the curve C2, having improved strain, with the strain position points designated by a square. The total strain experienced by the prior art sent section 530b is the area under the curve C2. Since both stent sections 530a and 530b experience the same expansion, the total strain is the same. That is to say, the area under the curve C1 is the same as the area under the curve C2.

It should be noted that the illustrated strains and loading are exemplary, and not meant to depict actual conditions or results. Instead, the illustrated strains are used for comparative purpose to demonstrate the effect of load on stent components having different geometries.

Turning to Figure 5C, the strain experienced by the prior art stent is relatively low at position points 1 and 2, reaching a strain of approximately 8 at the root of radial arc 510a (position point 2). The strain then increases dramatically to a maximum strain of approximately 50% at position point 3, i.e. the apex of radial arc 510a. The experienced strain is substantially symmetric about the apex of the radial arc 510, dramatically decreasing to a strain of approximately 8 at the root of the radial arc 510a (position point 4), and nearly 0% at the radial strut 508a, position point 5.

In comparison, the strain for the stent section 530b is relatively low at position points 1, but increases more uniformly between position points 2 and 3, reaching a strains of approximately 18% at the root of the radial arc 510b (position point 2) and 35% at the apex of radial arc 510b (position point 3). Similar to curve C1, curve C2 is substantially symmetric about position point 3.

As can be interpreted from Figures 5A through 5C, by modifying the material cross-section (adding or subtracting material) from the radial arc root (position points 2 and 4) the induced strain was increased. This decreases the induced strain at the radial arc apex (position point 3) since the total strain experienced by the section remains unchanged. Further, by modifying the cross-sectional area (adding or subtracting material) along the apex of radial arc 510b (position point 3), the induced strain is decreased. This automatically increases the induced strain at the radial arc 510b roots (position points 2 and 4). These modifications can be done individually as described, or in combination, iteratively, to develop a stent section 530b having improved near uniform strain along the radial arc 530b.

One advantage of having near uniform strain is that the peak strain (shown at position point 3) is greatly reduced. As a result, the stent may be expanded to a larger expansion diameter and still be within safe operating levels of induced strain. For example, the stent represented by curve C2 could be increased in diameter until the peak strain at position point 3 is increased from 35% to 50%.

The stent 300 according to one embodiment of the present invention is laser cut from a thin metallic tube having a substantially uniform wall thickness. To vary the cross-section of the stent components, particularly the radial arcs 310, the components have been tapered, with larger widths in areas of high loading to achieve near uniform stress and/or strain. It should be understood that the taper does not have to be uniform, which is to say of a consistently changing radius. Instead, the width of the radial arc 310 is dictated by the resultant stress and/or strain experienced by the radial arc 310 at various locations along its length.

Figures 3B through 3D show hoop elements 322 with tapered radial arcs 310 according to one embodiment of the present invention.

Turning to Figure 3B, a proximal hoop element 322a is shown according to one embodiment of the present invention. The hoop element 322a is comprised of two radial struts, 308a1 and 308a2, and two different radial arcs, 310a1 and 310a2. The radial struts 308a1 and 308a2 are shown having different profiles in the illustrated embodiment, but this should not be interpreted to limit the scope of the invention. Other embodiments may have identical or near identical radial strut profiles.

Radial arc 310a1 connects radial strut 308a2 to radial strut 308a1, and is not connected to flex connector 314. Because the radial arc 310a1 is not connected to the flex connector 314, the radial arc 310a1 experiences near proportioned loading, and thus has a substantially symmetrical geometry (with radial strut (308a1 or 308a2) connection points 315a having substantially equal cross-sections) to maintain near uniform stress and/or strain throughout. The approximate midpoint of the radial arc 310a1 according to the illustrated embodiment experiences slightly higher loading than the radial arc 310a1 connection points 315a. To accommodate the higher loading and maintain near uniform stress and/or strain throughout the radial arc 310a1, the midpoint of the radial arc 310a1 is thicker (has a greater width) than the radial arc to radial strut connection points 315a.

Conversely, radial arc 310a2 is directly connected to flex connector 314, and experiences unbalanced loading. To maintain substantially uniform stress and/or strain through the radial arc 310a2, the arc 310a2 has a substantially asymmetrical geometry, with radial strut (308a1, 308a2) connection points (313a, 317a) respectively, having substantially unequal cross-sections. Because the radial arc 310a2 to flex connector 314 connection point 317a has a large cross-section, the connection point 319a, located immediately adjacent thereto, may have a slightly smaller width to maintain substantially uniform stress and/or strain. The approximate midpoint of the radial arc 310a2 according to the illustrated embodiment experiences slightly higher loading than the radial arc 310a2 connection points 313a and 319a. To accommodate the higher loading and maintain near uniform stress and/or strain throughout the radial arc 310a2, the midpoint of the radial arc 310a2 is thicker (has a greater width) than the radial arc to radial strut connection points 313a and 319a.

Figure 3C shows an internal hoop element 322b according to one embodiment of the present invention. The hoop element 322b is comprised of radial struts, 308b1, and 308b2, and radial arcs 310b1 and 310b2. Each radial arc (310b1, 310b2) connects radial strut 308b1 to radial strut 308b2. Each radial arc (310b1, 310b2) is also connected to flex connector 314 near the connection point with radial strut 308b2. Because the radial hoop element 322b is substantially symmetrical, the radial arcs (310b1, 310b2) experiences near proportioned loading, and thus have substantially symmetrical geometry connection points 315b, 313b, and 319b (having substantially equal cross-sections) to maintain near uniform stress and/or strain. The approximate midpoints of the radial arcs 310b1, 310b2 according to the illustrated embodiment experience slightly higher loading than the radial arcs 310b1, 310b2 connection points 315b, 313b, and 319b. To accommodate the higher loading and maintain near uniform stress and/or strain throughout the radial arcs 310b1, 310b2, the midpoints of the radial arcs 310b1, 310b2 are thicker (have greater width) than the radial arc to radial strut connection points 315b, 313b, and 319b.

Figure 3D illustrates a distal hoop element 322c according to one embodiment of the present invention. As earlier described, the distal hoop element 322c is a mirror image of the proximal hoop element 322a shown in Figure 3B. As such, the loading and resultant geometry of the strut members are similar.

A distal hoop element 322c is shown according to one embodiment of the present invention. The hoop element 322c is comprised of two radial struts, 308c1 and 308c2 and two different radial arcs 310c1 and 310c2.

Radial arc 310c1 connects radial strut 308c2 to radial strut 308c1, and is not connected to flex connector 314. Because the radial arc 310c1 is not connected to the flex connector 314, the radial arc 310c1 experiences near proportioned loading, and thus has a substantially symmetrical geometry (with radial strut (308c1 or 308c2) connection points 315c having substantially equal cross-sections) to maintain near uniform stress and/or strain through. The approximate midpoint of the radial arc 310c1 according to the illustrated embodiment experiences slightly higher loading than the radial arc 310c1 connection points 315c. To accommodate the higher loading and maintain near uniform stress and/or strain throughout the radial arc 310c1, the midpoint of the radial arc 310c1 is thicker (has a greater,width) than the radial arc to radial strut connection points 315a.

Conversely, radial arc 310c2 is directly connected to flex connector 314, and experiences unbalanced loading. To maintain substantially uniform stress and/or strain through the radial arc 310c2, the arc 310c2 has a substantially asymmetrical geometry, with radial strut (308c1, 308c2) connection points (313c, 317c) respectively, having substantially unequal cross-sections. Because the radial arc 310c2 to flex connector 314 connection point 317c has a large cross-section, the connection point 319c, located immediately adjacent thereto, may have a slightly smaller width to maintain substantially uniform stress and/or strain. The approximate midpoint of the radial arc 310c2 according to the illustrated embodiment experiences slightly higher loading than the radial arc 310c2 connection points 313c and 319c. To accommodate the higher loading and maintain near uniform stress and/or strain throughout the radial arc 310c2, the midpoint of the radial arc 310c2 is thicker (has a greater width) than the radial arc to radial strut connection points 313c and 319c.

The stent design according to the present invention may also be optimized around minimizing maximum stress and/or strain to obtain a stent that has near uniform stress and/or strain at each point along the flex connectors 314. This design will provide a more flexible stent, having flex connector sections of smaller cross-section where the initial measured load and stress and/or strain were low. The aforementioned criteria (i.e. adding or removing cross-section) is applied to the flex connector 314 until the resultant stress and/or strain is nearly uniform.

The radial struts 308 experience relatively low stress and/or strain compared to the flex connectors 314 and radial arcs 310, so tapering of the struts 308 is typically not necessary to minimize maximum stress and/or strain for fatigue resistance. However, increasing the cross-section of the radial struts 308 as illustrated in Figures 3A through 3D makes the struts 308, and thus the stent 300, more radio-opaque. This enhances the visibility of the stent during fluoroscopic procedures. Increasing the cross-section of the struts 308 may also include shaping or adding a shape to the strut to increase the strut size. In one embodiment a bulge shape 309 is added to the stent strut 308. However, one of skill in the art would understand that the type of geometric shape added to the strut 308 is not meant to limit the scope of the invention.

In addition to the embodiments described above, therapeutic or pharmaceutic agents may be added to any component of the device during fabrication to treat any number of conditions. Having radial struts 308 with increased widths, added shapes, or gradually increasing profiles will allow the stent to carry more agent.

Therapeutic or pharmaceutic agents may be applied to the device, such as in the form of a drug or drug eluting layer, or surface treatment after the device has been formed. In a preferred embodiment, the therapeutic and pharmaceutic agents may include any one or more of the following: antiproliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP) II_{b}/IIIₐ inhibitors and vitronectin receptor antagonists; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes - dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetominophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blockers; nitric oxide donors; anti-sense oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

While a number of variations of the invention have been shown and described in detail, other modifications and methods of use contemplated within the scope of this invention will be readily apparent to those of skill in the art based upon this disclosure. It is contemplated that various combinations or sub combinations of the specific embodiments may be made and still fall within the scope of the invention. For example, the embodiments variously shown to be cardiac stents may be modified to treat other vessels or lumens in the body, in particular other regions of the body where vessels or lumen need to be supported. This may include, for example, the coronary, vascular, non-vascular and peripheral vessels and ducts. Accordingly, it should be understood that various applications, modifications and substitutions may be made within the scope of the following claims.

## Claims

1. A stent (300) comprising a plurality of flexible connectors (326) connecting adjacent hoop components (312), each flexible connector being formed, as a continuous series of substantially longitudinally oriented flexible strut members (316) and a plurality of flexible arc members (318) connecting adjacent flexible struts, wherein at least one flexible arc member has a tapered profile to achieve near-uniform strain distribution along the flexible arc when the flexible arc undergoes deformation and wherein each flexible connector is shaped so as to nest together into the circumferentially adjacent flexible connector.

2. The stent of claim 1, wherein each of the plurality of flexible connectors comprises three longitudinally oriented flexible struts being connected at each end by one of four flexible arc members, thereby forming an N-shaped flexible connector.

## Patentansprüche

1. Stent (300), umfassend eine Mehrzahl von flexiblen Verbindern (326), die benachbarte Reifenkomponenten (312) verbinden, wobei jeder flexible Verbinder als eine kontinuierliche Reihe im Wesentlichen in Längsrichtung ausgerichteter flexibler Stegelemente (316) und eine Mehrzahl von flexiblen Bogenelementen (318) gebildet ist, die benachbarte flexible Stege verbinden, wobei mindestens ein flexibles Bogenelement ein sich verjüngendes Profil hat, um eine nahezu einheitliche Dehnungsverteilung entlang dem flexiblen Bogen zu erzielen, wenn der flexible Bogen deformiert wird, und wobei jeder flexible Verbinder so geformt ist, dass er sich in den umfangsmäßig benachbarten flexiblen Verbinder einschachtelt.

2. Stent nach Anspruch 1, wobei jeder der Mehrzahl von flexiblen Verbindern drei in Längsrichtung ausgerichtete flexible Stege umfasst, die an jedem Ende durch eines von vier flexiblen Bogenelementen verbunden sind, wodurch ein N-förmiger flexibler Verbinder gebildet wird.

## Revendications

1. Endoprothèse (300) comprenant une pluralité de connecteurs flexibles (326) reliant des composants en boucles adjacents (312), chaque connecteur flexible étant formé sous la forme d'une série continue d'éléments d'entretoises flexibles orientés substantiellement longitudinalement (316) et d'une pluralité d'éléments d'arcs flexibles (318) reliant des entretoises flexibles adjacentes, au moins un élément d'arc flexible ayant un profil conique pour réaliser une répartition presque uniforme des contraintes le long de l'arc flexible lorsque l'arc flexible est soumis à une déformation et chaque connecteur flexible étant formé de manière à s'emboîter dans le connecteur flexible circonférentiellement adjacent.

2. Endoprothèse selon la revendication 1, dans laquelle chacun de la pluralité de connecteurs flexibles comprend trois entretoises flexibles orientées longitudinalement qui sont connectées à chaque extrémité par l'un de quatre éléments d'arcs flexibles pour ainsi former un connecteur flexible en forme de N.
